# EUROPEAN PATENT SPECIFICATION

(11) **EP 1 694 122 B1**
(45) Date of publication and mention of the grant of the patent: **12.09.2007**
(21) Application number: 04819647.1
(22) Date of filing: 02.12.2004
(51) Int. Cl.: A01N 37/52

(54) **N-ARYLHYDRAZONE DERIVATIVES FOR SEED TREATMENT**
N-ARYLHYDRAZONDERIVATE FÜR DIE SAATGUTBEHANDLUNG
DERIVES DE N-ARYLHYDRAZONE POUR LE TRAITMENT DE SEMENCES

(30) Priority: 04.12.2003 US 526609 P
(43) Date of publication of application: 30.08.2006
(73) Proprietor: BASF Aktiengesellschaft, 67056 Ludwigshafen (DE)
(72) Inventor: VON DEYN, Wolfgang, Neustadt (DE); OLOUOMI-SADEGHI, Hassan, Raleigh, NC 27614 (US); KUHN, David, G., Apex, NC 27502 (US); ARMES, Nigel, Raleigh, NC 27613 (US); COTTER, Henry, Van, Tuyl, Raleigh, NC 27613 (US); HICKS, Carol, Raleigh, NC 27612 (US)
(86) International application number: PCT/EP2004/013686
(87) International publication number: WO 2005/053401

(56) References cited:
- EP-A- 0 604 798
- DATABASE CA [Online] CHEMICAL ABSTRACTS SERVICE, COLUMBUS, OHIO, US; KUHN, D. G. ET AL KUHN, D. G. ET AL: "Cycloalkyl-substituted amidrazones: a novel class of insect control agents Cycloalkyl-substituted amidrazones: a novel class of insect control agents" XP002330111 retrieved from STN Database accession no. 1998:294837 & ACS SYMPOSIUM SERIES , 686(SYNTHESIS AND CHEMISTRY OF AGROCHEMICALS V), 185-193 CODEN: ACSMC8; ISSN: 0097-6156 ACS SYMPOSIUM SERIES , 686(SYNTHESIS AND CHEMISTRY OF AGROCHEMICALS V), 185-193 CODEN: ACSMC8; ISSN: 0097-6156, 1998, cited in the application
- DATABASE CA [Online] CHEMICAL ABSTRACTS SERVICE, COLUMBUS, OHIO, US; FURCH, J. A. ET AL FURCH, J. A. ET AL: "Amidrazones: a new class of coleopteran insecticides Amidrazones: a new class of coleopteran insecticides" XP002330112 retrieved from STN Database accession no. 1998:294826 & ACS SYMPOSIUM SERIES , 686(SYNTHESIS AND CHEMISTRY OF AGROCHEMICALS V), 178-184 CODEN: ACSMC8; ISSN: 0097-6156 ACS SYMPOSIUM SERIES , 686(SYNTHESIS AND CHEMISTRY OF AGROCHEMICALS V), 178-184 CODEN: ACSMC8; ISSN: 0097-6156, 1998, cited in the application

## Description

The present invention provides a method for the protection of seeds comprising contacting the seeds before sowing and/ or after pregermination with a compound of formula 1: 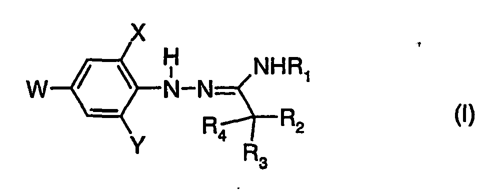 wherein
- W: is chlorine or trifluoromethyl;
- X and Y: are each independently chlorine or bromine;
- R¹: is C₁-C₆-alkyl, C₃-C₆-alkenyl, C₃-C₆-alkynyl, or C₃-C₆-cycloalkyl which may be substituted with 1 to 3 halogen atoms, or C₂-C₄-alkyl which is substituted by C₁-C₄-alkoxy;
- R² and R³: are C₁-C₆-alkyl or may be taken together to form C₃-C₆-cycloalkyl which may be unsubstituted or substituted by 1 to 3 halogen atoms;
- R⁴: is hydrogen or C₁-C₆-alkyl,
or the enantiomers or salts thereof,
in pesticidally effective amounts.

The invention also relates to seed comprising compounds of formula I.

The invention further relates to the use of compounds of formula I for the protection of seeds from soil pests.

One typical problem arising in the field of protection of seeds lies in the need to reduce the dosage rates of the active ingredient in order to reduce or avoid unfavorable environmental or toxicological effects whilst still allowing effective soil pest control.

Another problem encountered concerns the need to have available seed protection agents which are effective against a broad spectrum of soil pests.

There also exists the need for seed protection agents that combine know-down activity with prolonged control, that is, fast action with long lasting action.

Another difficulty in relation to the use of seed protection pesticides is that the repeated and exclusive application of an individual pesticidal compound leads in many cases to a rapid selection of soil pests which have developed natural or adapted resistance against the active compound in question. Therefore there is a need for seed protection agents that help prevent or overcome resistance.

It was therefore an object of the present invention to provide compounds which solve the problems of reducing the dosage rate and / or enhancing the spectrum of activity and / or combining know-down activity with prolonged control and / or to resistance management.

We have found that these objects are in part or in whole achieved by the compounds of formula I and compositions comprising them.

The insecticidal and acaricidal activity in plant protection in the agricultural field of some of the compounds of formula I has been described in EP-A 604 798, and also in J. A Furch et al., "Amidrazones: A New Class of Coleopteran Insecticides", ACS Symposium Series 686, Am. Chem. Soc., 1998, Chapter 18, p. 178 ff, and also in D. G. Kuhn et al., "Cycloalkyl-substituted Amidrazones: A Novel Class of Insect Control Agents", ACS Symposium Series 686, Am. Chem. Soc., 1998, Chapter 19, p. 185 ff.

Activity of compounds in plant protection against agricultural pests does not suggest their suitability for the protection of seeds which requires, for example, activity against soil pests, compatibility with the soil conditions (e.g. concerning binding of the compound to the soil), negligible phytotoxicity when applied to the seed, and appropriate movement to achieve necessary bioavailability (in soil or plant).

Surprisingly it has now been found that compounds of formula I are suitable for the protection of seeds.

The compounds of formula I can be prepared according to preparation methods described or referenced in EP-A 604 798 or modifications thereof.

In the definition of formula I shown above, the substituents have the following meanings:

"Halogen" will be taken to mean fluoro, chloro, bromo and iodo.

The term "alkyl" as used herein refers to a branched or unbranched saturated hydrocarbon group having 1 to 4 or 6 carbon atoms, especially C₁-C₆-alkyl such as methyl, ethyl, propyl, 1-methylethyl, butyl, 1-methylpropyl, 2-methylpropyl, 1,1-dimethylethyl, pentyl, 1-methylbutyl, 2-methylbutyl, 3-methylbutyl, 2,2-dimethylpropyl, 1-ethylpropyl, hexyl, 1,1-dimethylpropyl, 1,2-dimethylpropyl, 1-methylpentyl, 2-methylpentyl, 3-methylpentyl, 4-methylpentyl, 1,1-dimethylbutyl, 1,2-dimethylbutyl, 1,3-dimethylbutyl, 2,2-dimethylbutyl, 2,3-dimethylbutyl, 3,3-dimethylbutyl, 1-ethylbutyl, 2-ethylbutyl, 1,1,2-trimethylpropyl, 1,2,2-trimethylpropyl, 1-ethyl-1-methylpropyl and 1-ethyl-2-methylpropyl.

"Alkoxy" refers to a straight-chain or branched alkyl group having 1 to 4 carbon atoms (methyl, ethyl, propyl, 1-methylethyl, butyl, 1-methylpropyl, 2-methylpropyl, 1,1-dimethylethyl) bonded through an oxygen linkage, at any bond in the alkyl group. Examples include methoxy, ethoxy, propoxy, and isopropoxy.

"Cycloalkyl" refers to a monocyclic 3- to 6-membered saturated carbon atom ring, i.e. cyclopropyl, cyclobutyl, cyclopentyl, and cyclohexyl.

With respect to the intended use of the compounds of formula I, particular preference is given to the following meanings of the substituents, in each case on their own or in combination:

Preference is given to compounds of formula I wherein W is trifluoromethyl.

Preference is further given to compounds of formula I wherein X and Y are both chlorine.

Moreover, preferred are compounds of formula I wherein R¹ is C₁-C₆-alkyl, especially ethyl.

Preference is further given to compounds of formula I wherein R² and R³ are both methyl.

Moreover, preferred are compounds of formula I wherein R² and R³ form a cyclopropyl ring which is unsubstituted or substituted by 1 to 3 halogen atoms, especially chlorine and bromine.

Moreover, particularly preferred are compounds of formula I wherein R² and R³ form a cyclopropyl ring which is substituted by 2 halogen atoms.

Moreover, particularly preferred are compounds of formula I wherein R² and R³ form a cyclopropyl ring which is substituted by 2 chlorine atoms.

Particularly preferred are compounds of formula I wherein R² and R³ form a 2,2-dichlorocyclopropyl ring.

Preference is further given to compounds of formula I wherein R⁴ is C₁-C₆ alkyl, especially methyl.

Particularly preferred are compounds of formula I wherein R², R³ and R⁴are all methyl.

Moreover, particularly preferred are compounds of formula I wherein R², R³ and R⁴ form a moiety 1-methyl-2,2-dichlorocyctopropyl.

Preference is further given to compounds of formula I wherein
W is trifluoromethyl;
X and Y are each independently chlorine or bromine;
R¹ is C₁-C₆-alkyl;
R² and R³ are C₁-C₆-alkyl or may be taken together to form C₃-C₆-cycloalkyl which is substituted by 1 to 2 halogen atoms;
R⁴ is C₁-C₆-alkyl;
or the enantiomers or salts thereof.

Particular preference is given to N-ethyl-2,2-dimethylpropionamide-2-(2,6-dichloro-α,α,α-trifluoro-p-tolyl)hydrazone and N-Ethyl-2,2-dichloro-1-methylcyclopropane-carboxamide, 2-(2,6-dichloro- *α,α,α* -trifluoro-p-tolyl)hydrazone.

Furthermore, particular preference with respect to the use in the present invention is given to the compound of formula I-1 (N-ethyl-2,2-dimethylpropionamide-2-(2,6-dichloro- *α,α,α* -trifluoro-p-tolyl)-hydrazone):

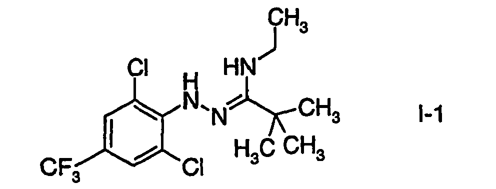

Moreover, particular preference with respect to the use in the present invention is given to the compound of formula I-2 (N-Ethyl-2,2-dichloro-1-methylcyclo-propanecarboxamide-2-(2,6-dichloro- α,α,α -tri-fluoro-p-tolyl)hydrazone):

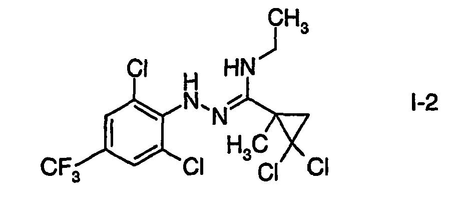

With respect to their use, particular preference is given to the compounds I-A compiled in the tables below. Moreover, the groups mentioned for a substituent in the tables are on their own, independently of the combination in which they are mentioned, a particularly preferred embodiment of the substituent in question.

With respect to their use, particular preference is also given to the hydrochloric acid, maleic acid, dimaleic acid, fumaric acid, difumaric acid, methane sulfenic acid, methane sulfonic acid, and succinic acid adducts of the compounds of the tables below.

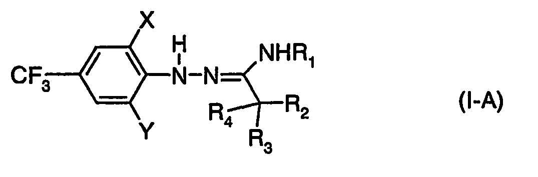

**Table A**

| **No.** | **R¹** | **R²** | **R³** | **R⁴** | **X** | **Y** |
|---|---|---|---|---|---|---|
| A-1 | CH₃ | 2,2-dichlorocyclopropyl | | H | Cl | Cl |
| A-2 | CH₃ | 2,2-dibromocyclopropyl | | H | Cl | Cl |
| A-3 | CH₃ | CH₃ | CH₃ | CH₃ | Cl | Cl |
| A-4 | CH₃ | CH₂CH₃ | CH₃ | CH₃ | Cl | Cl |
| A-5 | CH₃ | 2,2-dichlorocyclopropyl | | CH₃ | Cl | Cl |
| A-6 | CH₃ | 2,2-dibromocyclopropyl | | CH₃ | Cl | Cl |
| A-7 | CH₃ | 2,2-dichlorocyclopropyl | | H | Br | Br |
| A-8 | CH₃ | 2,2-dibromocyclopropyl | | H | Br | Br |
| A-9 | CH₃ | CH₃ | CH₃ | CH₃ | Br | Br |
| A-10 | CH₃ | CH₂CH₃ | CH₃ | CH₃ | Br | Br |
| A-11 | CH₃ | 2,2-dichlorocyclopropyl | | CH₃ | Br | Br |
| A-12 | CH₃ | 2,2-dibromocyclopropyl | | CH₃ | Br | Br |
| A-13 | CH₂CH₃ | 2,2-dichlorocyclopropyl | | H | Cl | Cl |
| A-14 | CH₂CH₃ | 2,2-dibromocyclopropyl | | H | Cl | Cl |
| A-15 | CH₂CH₃ | CH₃ | CH₃ | CH₃ | Cl | Cl |
| A-16 | CH₂CH₃ | CH₂CH₃ | CH₃ | CH₃ | Cl | Cl |
| A-17 | CH₂CH₃ | 2,2-dichlorocyclopropyl | | CH₃ | Cl | Cl |
| A-18 | CH₂CH₃ | 2,2-dibromocyclopropyl | | CH₃ | Cl | Cl |
| A-19 | CH₂CH₃ | 2,2-dichlorocyclopropyl | | H | Br | Br |
| A-20 | CH₂CH₃ | 2,2-dibromocyclopropyl | | H | Br | Br |
| A-21 | CH₂CH₃ | CH₃ | CH₃ | CH₃ | Br | Br |
| A-22 | CH₂CH₃ | CH₂CH₃ | CH₃ | CH₃ | Br | Br |
| A-23 | CH₂CH₃ | 2,2-dichlorocyclopropyl | | CH₃ | Br | Br |
| A-24 | CH₂CH₃ | 2,2-dibromocyclopropyl | | CH₃ | Br | Br |
| A-25 | CH₂CH₂CH₃ | 2,2-dichlorocyclopropyl | | H | Cl | Cl |
| A-26 | CH₂CH₂CH₃ | 2,2-dibromocyclopropyl | | H | Cl | Cl |
| A-27 | CH₂CH₂CH₃ | CH₃ | CH₃ | CH₃ | Cl | Cl |
| A-28 | CH₂CH₂CH₃ | CH₂CH₃ | CH₃ | CH₃ | Cl | Cl |
| A-29 | CH₂CH₂CH₃ | 2,2-dichlorocyclopropyl | | CH₃ | Cl | Cl |
| A-30 | CH₂CH₂CH₃ | 2,2-dibromocyclopropyl | | CH₃ | Cl | Cl |
| A-31 | CH₂CH₂CH₃ | 2,2-dichlorocyclopropyl | | H | Br | Br |
| A-32 | CH₂CH₂CH₃ | 2,2-dibromocyclopropyl | | H | Br | Br |
| A-33 | CH₂CH₂CH₃ | CH₃ | CH₃ | CH₃ | Br | Br |
| A-34 | CH₂CH₂CH₃ | CH₂CH₃ | CH₃ | CH₃ | Br | Br |
| A-35 | CH₂CH₂CH₃ | 2,2-dichlorocyclopropyl | | CH₃ | Br | Br |
| A-36 | CH₂CH₂CH₃ | 2,2-dibromocyclopropyl | | CH₃ | Br | Br |
| A-37 | C₃H₅ | 2,2-dichlorocyclopropyl | | H | Cl | Cl |
| A-38 | C₃Hₛ | 2,2-dibromocyclopropyl | | H | Cl | Cl |
| A-39 | C₃H₆ | CH₃ | CH₃ | CH₃ | Cl | Cl |
| A-40 | C₃H₆ | CH₂CH₃ | CH₃ | CH₃ | Cl | Cl |
| A-41 | C₃H₆ | 2,2-dichlorocyclopropyl | | CH₃ | Cl | Cl |
| A-42 | C₃H₅ | 2,2-dibromocyclopropyl | | CH₃ | Cl | Cl |
| A-43 | C₃H₅ | 2,2-dichlorocyclopropyl | | H | Br | Br |
| A-44 | C₃H₆ | 2,2-dibromocyclopropyl | | H | Br | Br |
| A-45 | C₃H₅ | CH₃ | CH₃ | CH₃ | Br | Br |
| A-46 | C₃H₅ | CH₂CH₃ | CH₃ | CH₃ | Br | Br |
| A-47 | C₃H₅ | 2,2-dichlorocyclopropyl | | CH₃ | Br | Br |
| A-48 | C₃H₅ | 2,2-dibromocyclopropyl | | CH₃ | Br | Br |
| A-49 | CH₂OCH₃ | 2,2-dichlorocyclopropyl | | H | Cl | Cl |
| A-50 | CH₂OCH₃ | 2,2-dibromocyclopropyl | | H | Cl | Cl |
| A-51 | CH₂OCH₃ | CH₃ | CH₃ | CH₃ | Cl | Cl |
| A-52 | CH₂OCH₃ | CH₂CH₃ | CH₃ | CH₃ | Cl | Cl |
| A-53 | CH₂OCH₃ | 2,2-dichlorocyclopropyl | | CH₃ | Cl | Cl |
| A-54 | CH₂OCH₃ | 2,2-dibromocyclopropyl | | CH₃ | Cl | Cl |
| A-55 | CH₂OCH₃ | 2,2-dichlorocyclopropyl | | H | Br | Br |
| A-56 | CH₂OCH₃ | 2,2-dibromocyclopropyl | | H | Br | Br |
| A-57 | CH₂OCH₃ | CH₃ | CH₃ | CH₃ | Br | Br |
| A-58 | CH₂OCH₃ | CH₂CH₃ | CH₃ | CH₃ | Br | Br |
| A-59 | CH₂OCH₃ | 2,2-dichlorocyclopropyl | | CH₃ | Br | Br |
| A-60 | CH₂OCH₃ | 2,2-dibromocyclopropyl | | CH₃ | Br | Br |
| A-61 | CH₂OCH₂CH₃ | 2,2-dichlorocyclopropyl | | H | Cl | Cl |
| A-62 | CH₂OCH₂CH₃ | 2,2-dibromocyclopropyl | | H | Cl | Cl |
| A-63 | CH₂OCH₂CH₃ | CH₃ | CH₃ | CH₃ | Cl | Cl |
| A-64 | CH₂OCH₂CH₃ | CH₂CH₃ | CH₃ | CH₃ | Cl | Cl |
| A-65 | CH₂OCH₂CH₃ | 2,2-dichlorocyclopropyl | | CH₃ | Cl | Cl |
| A-66 | CH₂OCH₂CH₃ | 2,2-dibromocyclopropyl | | CH₃ | Cl | Cl |
| A-67 | CH₂OCH₂CH₃ | 2,2-dichlorocyclopropyl | | H | Br | Br |
| A-68 | CH₂OCH₂CH₃ | 2,2-dibromocyclopropyl | | H | Br | Br |
| A-69 | CH₂OCH₂CH₃ | CH₃ | CH₃ | CH₃ | Br | Br |
| A-70 | CH₂OCH₂CH₃ | CH₂CH₃ | CH₃ | CH₃ | Br | Br |
| A-71 | CH₂OCH₂CH₃ | 2,2-dichlorocyclopropyl | | CH₃ | Br | Br |
| A-72 | CH₂OCH₂CH₃ | 2,2-dibromocyclopropyl | | CH₃ | Br | Br |

The compounds of the formula I are suitable for the protection of seeds from soil pests, especially from those selected from the following list:
millipedes (Diplopoda), hemiptera (homoptera and heteroptera), Orthoptera,
lepidopterans (Lepidoptera), for example *Agrotis ipsilon, Agrotis segetum, Chilo ssp., Euxoa ssp., Momphidae, Ostrinia nubilalis, and Phthorimaea operculella,*
beetles (Coleoptera), for example *Agriotes lineatus, Agriotes obscurus, Aphthona euphoridae, Athous haemorrhoidalis, Atomaria linearis, Cetonia aurata; Ceuthorrhynchus assimilis, Ceuthorrhynchus napi, Chaetocnema tibialis, Ctenicera ssp., Diabrotica longicornis, Diabrotica speciosa, Diabrotica semi-punctata, Diabrotica virgifera, Limonius californicus, Melanotus communis, Otiorrhynchus ovatus, Phyllobius pyri, Phyllophaga sp., Phyllophaga cuyabana, Phyllophaga triticophaga, Phyllopertha horticola, Phyllotreta nemorum, Phyllotreta striolata, Popillia japonica, Sitona lineatus* and *Sitophilus granaria,*
flies (Diptera), for example *Chrysomya bezziana, Chrysomya hominivorax, Chrysomya macellaria, Contarinia sorghicola, Cordylobia anthropophaga, Dacus cucurbitae, Dacus oleae, Dasineura brassicae, Delia antique, Delia coarctata, Delia platura, Delia radicum, Fannia canicularis, Gasterophilus intestinalis, Geomyza Tripunctata, Glossina morsitans, Haematobia irritans, Haplodiplosis equestris, Hypoderma lineata; Lucilia caprina, Lucilia cuprina, Lucilia sericata, Lycoria pectoralis, Mayetiola destructor, Muscina stabulans, Oestrus ovis, Opomyza florum, Oscinella frit, Pegomya hysocyami, Phorbia antiqua, Phorbia brassicae, Phorbia coarctata, Psila rosae, Rhagoletis cerasi, Rhagoletis pomonella, Tabanus bovinus, Tipula oleracea* and *Tipula paludosa,*
thrips (Thysanoptera), e.g. Thrips simplex,
ants (Hymenoptera), e.g. *Atta capiguara, Atta cephalotes, Atta laevigata, Atta robusta, Atta sexdens, Atta texana, Monomorium pharaonis, Solenopsis geminata* and *Solenopsis invicta, Pogonomyrmex ssp.* and *Pheidole megacephala,*
termites (Isoptera), *e.g. Coptotermes ssp,*
springtails (Collembola), *e.g. Onychiurus ssp.*

In particular, the inventive mixtures are suitable for combating pests of the orders Diptera, Coleoptera, Lepidoptera, and Isoptera.

Moreover, the use of the compounds of formula I and compositions containing them for combating pests of the Diptera order, especially *Delia* species is especially preferred.

The use of the compounds of formula I and compositions containing them for combating pests of the Coleoptera order, especially *Diabrotica* species and *Elateridae* (wire-worms) is a further preferred embodiment of the present invention.

Furthermore, the use of the compounds of formula I and compositions containing them for combating pests of the Isoptera order is especially preferred.

The compounds of formula I can be used for the protection of the seed and the seedlings' roots and shoots against soil pests.

The protection of seed is preferred.

Especially preferred is the protection of seeds from the group of cereals (e.g. wheat, barley, rye), canola, sugar beet, maize, sorghum, sunflower, cotton, rice, peas, colza, potato, and market-garden crops like rice, wheat, barley, or rye.

The compounds of formula I are effective through both direct and indirect contact and ingestion, and also through trophallaxis and transfer.

Conventional seed treatment formulations include for example flowable concentrates FS, solutions LS, powders for dry treatment DS, water dispersible powders WS or granules for slurry treatment, water soluble powders SS and emulsion ES. Application to the seeds is carried out before sowing, either directly on the seeds or after having pregerminated the latter.

For use according to the present invention, the compounds I can be converted into the customary formulations, e.g. solutions, emulsions, suspensions, dusts, powders, pastes and granules. The use form depends on the particular purpose; it is intended to ensure in each case a fine and uniform distribution of the compound on the seed according to the invention.

The formulations are prepared in a known manner, e.g. by extending the active ingredient with solvents and/or carriers, if desired using emulsifiers and dispersants. Solvents/auxiliaries, which are suitable, are essentially:
- water, aromatic solvents (for example Solvesso products, xylene), paraffins (for example mineral fractions), alcohols (for example methanol, butanol, pentanol, benzyl alcohol), ketones (for example cyclohexanone, gamma-butyrolactone), pyrrolidones (NMP, NOP), acetates (glycol diacetate), glycols, fatty acid dimethylamides, fatty acids and fatty acid esters. In principle, solvent mixtures may also be used.
- carriers such as ground natural minerals (e.g. kaolins, clays, talc, chalk) and ground synthetic minerals (e.g. highly disperse silica, silicates); emulsifiers such as nonionic and anionic emulsifiers (e.g. polyoxyethylene fatty alcohol ethers, alkylsulfonates and arylsulfonates) and dispersants such as lignin-sulfite waste liquors and methylcellulose.

Suitable surfactants are alkali metal, alkaline earth metal and ammonium salts of lignosulfonic acid, naphthalenesulfonic acid, phenolsulfonic acid, dibutylnaphthalene-sulfonic acid, alkylarylsulfonates, alkyl sulfates, alkylsulfonates, fatty alcohol sulfates, fatty acids and sulfated fatty alcohol glycol ethers, furthermore condensates of sulfonated naphthalene and naphthalene derivatives with formaldehyde, condensates of naphthalene or of naphthalenesulfonic acid with phenol, octylphenol, nonylphenol, alkylphenyl polyglycol ethers, tributylphenyl polyglycol ether, tristearylphenyl polyglycol ether, alkylaryl polyether alcohols, alcohol and fatty alcohol/ethylene oxide condensates, ethoxylated castor oil, polyoxyethylene alkyl ethers, ethoxylated polyoxypropylene, lauryl alcohol polyglycol ether acetal, sorbitol esters, lignin-sulfite waste liquors and methylcellulose and ethylene oxide / propylene oxide block copolymers.

Substances which are suitable for the preparation of directly sprayable solutions, emulsions, pastes or oil dispersions are mineral oil fractions of medium to high boiling point, such as kerosene or diesel oil, furthermore coal tar oils and oils of vegetable or animal origin, aliphatic, cyclic and aromatic hydrocarbons, for example toluene, xylene, paraffin, tetrahydronaphthalene, alkylated naphthalenes or their derivatives, methanol, ethanol, propanol, butanol, cyclohexanol, cyclohexanone, isophorone, strongly polar solvents, for example dimethyl sulfoxide, N-methylpyrrolidone and water.

Powders, materials for spreading and dusts can be prepared by mixing or concomitantly grinding the active substances with a solid carrier.

Granules, for example coated granules, impregnated granules and homogeneous granules, can be prepared by binding the active ingredients to solid carriers. Examples of solid carriers are mineral earths such as silica gels, silicates, talc, kaolin, attaclay, limestone, lime, chalk, bole, loess, clay, dolomite, diatomaceous earth, calcium sulfate, magnesium sulfate, magnesium oxide, ground synthetic materials, fertilizers, such as, for example, ammonium sulfate, ammonium phosphate, ammonium nitrate, ureas, and products of vegetable origin, such as cereal meal, tree bark meal, wood meal and nutshell meal, cellulose powders and other solid carriers.

Stickers / adhesion agents can be added to improve the adhesion of the active materials on the seeds after treatment. Suitable adhesives are block copolymers EO/PO surfactants but also polyvinylalcoholsl, polyvinylpyrrolidones, polyacrylates, polymethacrylates, polybutenes, polyisobutylenes, polystyrene, polyethyleneamines, polyethyleneamides, polyethyleneimines (Lupasol®, Polymin®), polyethers, polyurethans and copolymers derived from these polymers.

In general, the formulations comprise from 0.01 to 95% by weight, preferably from 0.1 to 90% by weight, of the active ingredient. The active ingredients are employed in a purity of from 90% to 100%, preferably 95% to 100% (according to NMR spectrum).

The following are examples of formulations: 1. Products for direct application or for application after dilution with water

### A) Soluble concentrates (LS)

10 parts by weight of the active compounds are dissolved in water or in a water-soluble solvent. As an alternative, wetters or other auxiliaries are added. The active compound dissolves upon dilution with water.

### B) Dispersible concentrates (DC)

20 parts by weight of the active compounds are dissolved in cyclohexanone with addition of a dispersant, for example polyvinylpyrrolidone. Dilution with water gives a dispersion.

### C) Emulsifiable concentrates (EC)

15 parts by weight of the active compounds are dissolved in xylene with addition of calcium dodecylbenzenesulfonate and castor oil ethoxylate (in each case 5% strength). Dilution with water gives an emulsion.

### D) Emulsions (ES)

40 parts by weight of the active compounds are dissolved in xylene with addition of calcium dodecylbenzenesulfonate and castor oil ethoxylate (in each case 5% strength). This mixture is introduced into water by means of an emulsifier (Ultraturax) and made into a homogeneous emulsion. Dilution with water gives an emulsion.

### E) Suspensions (FS)

In an agitated ball mill, 20 parts by weight of the active compounds are comminuted with addition of dispersant, wetters and water or an organic solvent to give a fine active compound suspension. Dilution with water gives a stable suspension of the active compound.

### F) Water-dispersible granules and water-soluble granules (WG, SG)

50 parts by weight of the active compounds are ground finely with addition of dispersants and wetters and made into water-dispersible or water-soluble granules by means of technical appliances (for example extrusion, spray tower, fluidized bed). Dilution with water gives a stable dispersion or solution of the active compound.

### G) Water-dispersible powders and water-soluble powders (SS, WS)

75 parts by weight of the active compounds are ground in a rotor-stator mill with addition of dispersant, wetters and silica gel. Dilution with water gives a stable dispersion or solution with the active compound.

### 2. Products to be applied undiluted

### H) Dustable powders (DS)

5 parts by weight of the active compounds are ground finely and mixed intimately with 95% of finely divided kaolin. This gives a dustable product.

### I) Granules (GR, FG, GG, MG)

0.5 part by weight of the active compounds is ground finely and associated with 95.5% carriers. Current methods are extrusion, spray-drying or the fluidized bed. This gives granules to be applied undiluted.

The active ingredients can be used as such, in the form of their formulations or the use forms prepared therefrom, eg. in the form of directly sprayable solutions, powders, gels, suspensions or dispersions, emulsions, oil dispersions, pastes, dustable products, materials for spreading, or granules, microcapsules (CS), pellets or tablets, by means of spraying, atomizing, dusting, spreading or pouring. The use forms depend entirely on the intended purposes; it is intended to ensure in each case the finest possible distribution of the active ingredients according to the invention.

Aqueous use forms can be prepared from emulsion concentrates, pastes or wettable powders (sprayable powders, oil dispersions) by adding water. To prepare emulsions, pastes or oil dispersions, the substances, as such or dissolved in an oil or solvent, can be homogenized in water by means of a wetter, tackifier, dispersant or emulsifier. Alternatively, it is possible to prepare concentrates composed of active substance, wetter, tackifier, dispersant or emulsifier and, if appropriate, solvent or oil, and such concentrates are suitable for dilution with water.

The active ingredient concentrations in the ready-to-use products can be varied within relatively wide ranges. In general, they are from 0.01 to 80%, preferably from 0.1 to 50%.

Various types of oils, wetters, adjuvants, herbicides, fungicides, other pesticides, or bactericides may be added to the active ingredients, if appropriate just immediately prior to use. These agents usually are admixed with the agents according to the invention in a weight ratio of 1:100 to 100:1.

Preferred are FS formulations.

Preferred FS formulations of compounds of formula I for seed treatment comprise from 0.5 to 80% of the active ingredient, from 0,05 to 5 % of a wetter, from 0.5 to 15 % of a dispersing agent, from 0,1 to 5 % of a thickener, from 5 to 20 % of an anti-freeze agent, from 0,1 to 2 % of an anti-foam agent, from 1 to 20 % of a pigment and/or a dye, from 0 to 15 % of a sticker /adhesion agent, from 0 to 75 % of a filler/vehicle, and from 0,01 to 1 % of a preservative.

In the treatment of seed, the application rates of the mixture are generally from 0,1 g to 10 kg per 100 kg of seed, preferably from 1 g to 5 kg per 100 kg of seed, in particular from 1 g to 200 g per 100 kg of seed.

In the control of pests, the application of the compound of formula I or of the composition comprising it is carried out by spraying or dusting the seeds or the soil (and thereby the seeds) after sowing.

A further subject of the invention is a method of treating the seed in the seed drill with a granular formulation containing the active ingredient or a composition comprising it, with optionally one or more solid or liquid, agriculturally acceptable carriers and/or optionally with one or more agriculturally acceptable surfactants. This method is advantageously employed in seedbeds of cereal, maize, cotton and sunflower. For cereals and maize, the rates for compounds of formula I are between 50 and 500 g/ha.

The invention also relates to the propagation product of plants, and especially the treated seed comprising, that is, coated with and/or containing, a compound of formula I or a composition comprising it. The term "coated with and/or containing" generally signifies that the active ingredient is for the most part on the surface of the propagation product at the time of application, although a greater or lesser part of the ingredient may penetrate into the propagation product, depending on the method of application. When the said propagation product is (re)planted, it may absorb the active ingredient.

The seed comprises the inventive mixtures in an amount of from 0,1 g to 10 kg per 100 kg of seed.

The following list of pesticides together with which the compounds according to the invention can be used, is intended to illustrate the possible combinations, but not to impose any limitation:
Organophosphates: Acephate, Azinphos-methyl, Chlorpyrifos, Chlorfenvinphos, Diazinon, Dichlorvos, Dicrotophos, Dimethoate, Disulfoton, Ethion, Fenitrothion, Fenthion, Isoxathion, Malathion, Methamidophos, Methidathion, Methyl-Parathion, Mevinphos, Monocrotophos, Oxydemeton-methyl, Paraoxon, Parathion, Phenthoate, Phosalone, Phosmet, Phosphamidon, Phorate, Phoxim, Pirimiphos-methyl, Profenofos, Prothiofos, Sulprophos, Terbufos, Triazophos, Trichlorfon;
Carbamates: Alanycarb, Benfuracarb, Carbaryl, Carbosulfan, Fenoxycarb, Furathiocarb, Indoxacarb, Methiocarb, Methomyl, Oxamyl, Pirimicarb, Propoxur, Thiodicarb, Triazamate;
Pyrethroids: Bifenthrin, Cyfluthrin, Cypermethrin, alpha-Cypermethrin, Deltamethrin, Esfenvalerate, Ethofenprox, Fenpropathrin, Fenvalerate, Cyhalothrin, Lambda-Cyhalothrin, Permethrin, Silafluofen, Tau-Fluvalinate, Tefluthrin, Tralomethrin, Zeta-Cypermethrin;
Arthropod growth regulators: a) chitin synthesis inhibitors: benzoylureas: Chlorfluazuron, Diflubenzuron, Flucycloxuron, Flufenoxuron, Hexaflumuron, Lufenuron, Novaluron, Teflubenzuron, Triflumuron; Buprofezin, Diofenolan, Hexythiazox, Etoxazole, Clofentazine; b) ecdysone antagonists: Halofenozide, Methoxyfenozide, Tebufenozide; c) juvenoids: Pyriproxyfen, Methoprene, Fenoxycarb; d) lipid biosynthesis inhibitors: Spirodiclofen;
Neonicotinoids: Acetamiprid, Clothianidin, Flonicamid, lmidacloprid, Nitenpyram, Thiacloprid, Thiamethoxam;
Various: Abamectin, Acequinocyl, Amitraz, Azadirachtin, Bifenazate, *Bacillus thuringiensis, Bacillus subtilis,* Cartap, Chlorfenapyr, Chlordimeform, Cyromazine, Diafenthiuron, Dinetofuran, Diofenolan, Emamectin, Endosulfan, Ethiprole, Fenazaquin, Fipronil, Formetanate, Formetanate hydrochloride, Hydramethylnon, Indoxacarb, 4-{(2Z)-2-({[4-(trifluoro-methoxy)anilino] carbonyl} hydrazono)-2-[3-(trifluoromethyl)-phenyl]ethyl} benzo-nitrile, Pyridaben, Pymetrozine, Spinosad, Sulfur, Tebufenpyrad, and Thiocyclam.

In general, "pesticidally effective amount" means the amount of active ingredient needed to achieve an observable effect on growth, including the effects of necrosis, death, retardation, prevention, and removal, destruction, or otherwise diminishing the occurrence and activity of the target organism. The pesticidally effective amount can vary for the various compounds/compositions used in the invention. A pesticidally effective amount of the compositions will also vary according to the prevailing conditions such as desired pesticidal effect and duration, weather, target species, locus, mode of application, and the like.

The pesticidal action of the compounds is demonstrated by the following experiments:
Corn Rootworm, Wireworm and Seed Corn Maggot Seed Treatment Assay
Protection of seeds from southern corn rootworm, *Diabrotica undecimpunctata howardi* (larvae), wireworm, *Agriotis lineatus* (larvae), and seed corn maggot, *Delia platura* (eggs)

The active compounds were formulated in a solvent-surfactant carrier consisting of 15% acetone and 0.05% Tween 20^{™} (Polyoxyethylene sorbitan monolaureate) in water.

Corn seeds (corn Truckers Favorite') were treated in lots of 50 by shaking with 450 µl of the compound preparation in a glass jar and were dried. Seeds were planted one per 120 ml container with air holes in the cap in sandy loam. Ten insect eggs or larvae were added to each container. Egg or larvae mortality and feeding damage to roots and shoots were evaluated seven days after planting.

Percent mortality of the insect and feeding damage are calculated compared to the blank solvent-surfactant control.

At 251 g active ingredient per 100 kg of seed, compounds I-1 and I-2 provided over 30% protection of seed and seedling from wireworm feeding damage.

## Claims

1. A method for the protection of seed comprising contacting the seeds before sowing and/or after pregermination with a compound of formula I 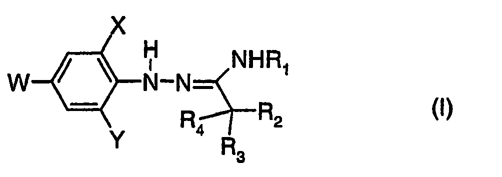 wherein
W is chlorine or trifluoromethyl;
X and Y are each independently chlorine or bromine;
R¹ is C₁-C₆-alkyl, C₃-C₆-alkenyl, C₃-C₆-alkynyl, or
C₃-C₆-cycloalkyl which may be substituted with 1 to 3 halogen atoms, or C₂-C₄-alkyl which is substituted by C₁-C₄-alkoxy;
R² and R³ are C₁-C₆-alkyl or may be taken together to form C₃-C₆-cycloalkyl which may be unsubstituted or substituted by 1 to 3 halogen atoms;
R⁴ is hydrogen or C₁-C₆-alkyl,
or the enantiomers or salts thereof,
in pesticidally effective amounts.

2. The method according to claim 1 wherein the compound of formula I is applied in an amount of from 0,1 g to 10 kg per 100 kg of seeds.

3. The method according to claim 1 wherein the compound of formula I is applied in an amount of from 1 g to 2 g per 100 kg of seeds.

4. The method according to claims 1 to 3 wherein the compound of formula I is a compound of formula I-1.

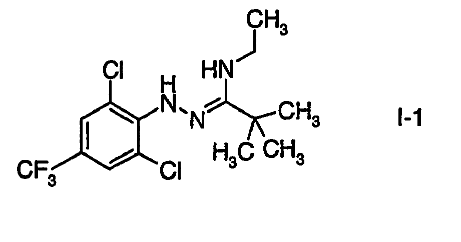

5. The method according to claims 1 to 3 wherein the compound of formula I is a compound of formula I-2.

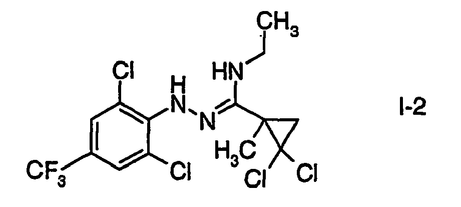

6. A method for the protection of seeds from soil pests of the Diptera, Lepidoptera, Coleoptera and Isoptera orders.

7. The use of the compounds of formula I as defined in claims 1, 4 or 5 for the protection of seeds from soil pests.

8. Seed, comprising the compound of formula I as defined in claims 1,4 or 5 in an amount of from 0,1 g to 10 kg per 100 kg of seed.

## Patentansprüche

1. Verfahren zum Schutz von Saatgut, bei dem man das Saatgut vor dem Säen und/oder nach dem Vorkeimen mit einer Verbindung der Formel I 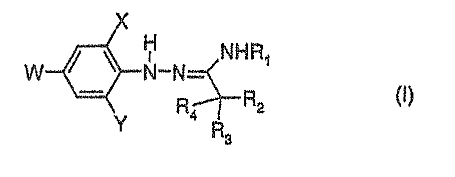 in der
W Chlor oder Trifluormethyl bedeutet,
X und Y jeweils unabhängig Chlor oder Brom bedeuten,
R¹ C₁-C₆-Alkyl, C₃-C₆-Alkenyl, C₃-C₆-Alkinyl oder C₃-C₆-Cycloalkyl, das durch 1 bis 3 Halogenatome substituiert sein kann, oder C₂-C₄-Alkyl, das durch C₁-C₄-Alkoxy substituiert ist, bedeutet,
R² und R³ C₁-C₆-Alkyl bedeuten oder gemeinsam C₃-C₆-Cycloalkyl, das unsubstituiert oder durch 1 bis 3 Halogenatome substituiert sein kann, bilden können,
R⁴ Wasserstoff oder C₁-C₆-Alkyl bedeutet,
oder ihren Enantiomeren oder Salzen
in pestizidwirksamen Mengen behandelt.

2. Verfahren nach Anspruch 1, wobei die Verbindung der Formel I in einer Menge von 0,1 g bis 10 kg pro 100 kg Saatgut eingesetzt wird.

3. Verfahren nach Anspruch 1, wobei die Verbindung der Formel I in einer Menge von 1 g bis 2 g pro 100 kg Saatgut eingesetzt wird.

4. Verfahren nach den Ansprüchen 1 bis 3, wobei es sich bei der Verbindung der Formel I um eine Verbindung der Formel I-1 handelt.

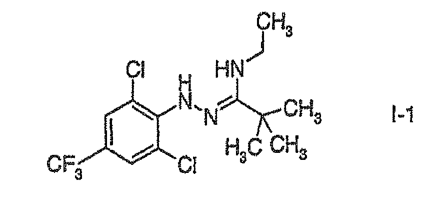

5. verfahren nach den Ansprüchen 1 bis 3, wobei es sich bei der Verbindung der Formel I um eine Verbindung der Formel I-2 handelt.

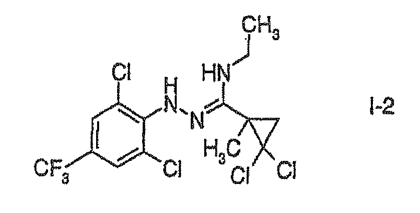

6. Verfahren zum Schützen von Saatgut gegen Bodeninsekten der Ordnungen Diptera, Lepidoptera, Coleoptera und Isoptera.

7. Verwendung der Verbindungen der Formel I nach den Ansprüchen 1, 4 oder 5 zum Schützen von Samen gegen Bodeninsekten.

8. Saatgut, enthaltend die Verbindung der Formel I gemäß den Ansprüchen 1, 4 oder 5 in einer Menge von 0,1 g bis 10 kg pro 100 kg Saatgut.

## Revendications

1. Procédé de protection de semences comprenant la mise en contact des semences, avant le semis et/ou après la prégermination, avec un composé de la formule I 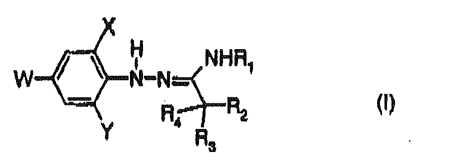 dans laquelle .
W représente du chlore ou un trifluorométhyle,
X et Y représentent chacun indépendamment l'un de l'autre du chlore ou du brome,
R¹ représente un alkyle en C₁ à C₆, un alcényle en C₃ à C₆, un alcynyle en C₃ à C₆ ou un cycloalkyle en C₃ à C₆ qui peut être substitué par 1 à 3 atomes d'halogène, ou un alkyle en C₂ à C₄ substitué par un alcoxy en C₁ à C₄,
R² et R³ représentent un alkyle en C₁ à C₆ ou peuvent être groupés pour former un cycloalkyle en C₃ à C₆ qui peut être non substitué ou substitué par 1 à 3 atomes d'halogène,
R⁴ représente de l'hydrogène ou un alkyle en C₁ à C₆,
ou les énantiomères ou les sels de ceux-ci,
en des quantités efficacement pesticides.

2. Procédé suivant la revendication 1, dans lequel le composé de la formule I est appliqué en une quantité de 0,1 g à 10 kg par 100 kg de semences.

3. Procédé suivant la revendication 1, dans lequel le composé de la formule I est appliqué en une quantité de 1 g à 2 g par 100 kg de semences.

4. Procédé suivant les revendications 1 à 3, dans lequel le composé de la formule I est un composé de la formule I-1

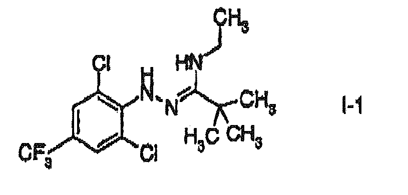

5. Procédé suivant les revendications 1 à 3, dans lequel le composé de la formule I est un composé de la formule 1-2.

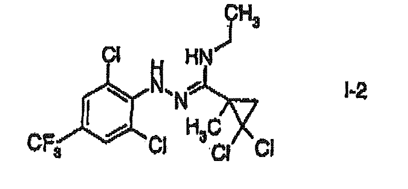

6. Procédé de protection de semences contre des parasites du sol des ordres des diptères, des lépidoptères, des coléoptères et des isoptères.

7. Utilisation des composés de la formule I tels que définis dans les revendications 1, 4 ou 5 pour la protection de semences contre des parasites du sol.

8. Semence, comprenant le composé de la formule I tel que défini dans les revendications 1, 4 ou 5 en une quantité de 0,1 g à 10 kg par 100 kg de semences.
